# EUROPEAN PATENT SPECIFICATION

(11) **EP 2 086 413 B1**
(45) Date of publication and mention of the grant of the patent: **24.07.2013**
(21) Application number: 06828930.5
(22) Date of filing: 07.11.2006
(51) Int. Cl.: A61B 6/04, A61G 13/04, F16M 11/12

(54) **ROTATION MECHANISM**
DREHMECHANISMUS
MÉCANISME DE ROTATION

(43) Date of publication of application: 12.08.2009
(73) Proprietor: Elekta AB (PUBL), 103 93 Stockholm (SE)
(72) Inventor: BOURNE, Duncan Neil, Redhill Surrey RH1 3BA (GB)
(74) Representative: Hall, Christopher David
(86) International application number: PCT/EP2006/010625
(87) International publication number: WO 2008/055517

(56) References cited:
- WO-A-97/42876
- US-B1- 6 182 582
- US-B1- 6 416 219

## Description

### FIELD OF THE INVENTION

The present invention relates to a rotation mechanism. It seeks to provide a mechanism allowing up to three degrees of rotational adjustment in a compact and space-efficient arrangement.

### BACKGROUND ART

A generic displacement applied to an object in three dimensional space includes a total of six degrees of freedom - three translational degrees of freedom and three rotational degrees of freedom in respect of each of the three axes. In order to place an object in space at an arbitrary location and orientation, an articulation system is therefore required that has at least six degrees of freedom.

The three translational degrees of freedom are generally straightforward to provide. A wide range of mechanisms exist that are able to do so, including rack and pinion arrangements, belt-driven systems, worm gears and the like. Difficulty can arise in allowing for the three rotational degrees of freedom, especially where space is limited.

One such example is a couch for a patient, such as a patient undergoing radiotherapy. The therapeutic beam of radiation must be positioned precisely relative to the patient, but the apparatus for producing the beam typically weights several tonnes and must itself be rotated around the patient as part of the treatment. Accordingly, for alignment purposes it is easier to move the patient than to move the apparatus. This means that the couch must include a mechanism able to move the patient in all six degrees of freedom.

Generally, the amplitude of rotational movement that is clinically required is not excessive, since the patient's prone position is usually largely the same and all that is required is fine-tuning so that the position during treatment is aligned to the position during previous investigative procedures. A large range of vertical adjustment will be useful in terms of patient comfort, to ease transfer of the patient from a standing position alongside the couch, a sitting position in a wheelchair, or a prone position on a bed. A large longitudinal movement may be required for certain forms of treatment, such as helical scan treatment planes which require a continuous movement of the patient during a rotational movement of the radiation source. Lateral movement is usually confined to fine-tuning.

This mechanism needs to be accommodated beneath the couch, at one end thereof. Clearly, the space above the couch will be occupied by the patient, and the space either side of the couch will be needed for access. However, some space under one end of the couch is also needed so that the radiation source can pass beneath the patient. Such sources direct the beam towards the patient from a number of different directions, or from a continuous sweeping arc. This will include directing the beam from beneath the patient, and therefore the couch needs to allow access from beneath, at least at the end proximate the source. This can present difficulties in the design of a mechanism to allow rotational adjustment.

US 6,416,219 discloses a treatment-diagnostic apparatus with a positioning device with a positioning plate for the treatment and/or examination of a subject that is adjustable at a base and is transparent for radiation in at least one region. The positioning plate, with reference to the base, is mounted so as to be adjustable at least along its longitudinal axis and is mounted at the base so as to be adjustable in height, as well as around three spatial axes x, y, z.

### SUMMARY OF THE INVENTION

The present invention therefore provides a patient support as defined in the claims appended hereto.

Wedge elements are placed between the rotateable elements in order to place their axes in a non-parallel arrangement. Since the rotation axes of the rotateable elements are not parallel, as one or more elements are rotated, the wedge elements that lie between them (or effectively lie between them) can be brought into and out of alignment. When aligned oppositely, the angles of the wedge elements will cancel out and no or a minimum total angle will result. When aligned together, the angles of the wedge elements will add and a maximum total angle will result.

Thus, a rotateable element between the wedges (or effective wedges) allow a variable angle to be created by the mechanism between its top and bottom faces. The use of a second rotateable element allows that angle to be oriented in any chosen direction. The use of a third rotateable element allows for any accumulated rotation to be cancelled out.

The rotateable elements are generally disc-shaped, such as in the form of torque motors. Other sources of rotation could be used, such as a conventional motor and gearbox, but a torque motor is likely to be the most elegant and compact solution.

There is a control apparatus for controlling the first, second and third rotateable elements to each rotate by a specific angle of rotation. This rotates one of the first, second and third rotateable elements by an angle substantially equal to the complement of the sum of the angles through which the remaining two of the first, second and third rotateable elements have been rotated, to cancel out the accumulated rotation of the remaining two and return any apparatus supported by the mechanism to its original alignment. It is to be preferred that the control apparatus does so by rotating that one of the first, second and third rotateable elements at a rate equal to the total rate of angular rotation of the remaining two of the first, second and third rotateable elements but in an opposite direction. This prevents transients in the angular position of any such apparatus.

### BRIEF DESCRIPTION OF THE DRAWINGS

An embodiment of the present invention will now be described by way of example, with reference to the accompanying figures in which;
Figure 1 shows a first embodiment of a patient table according to the present invention;
Figure 2 shows a second embodiment of a patient table according to the present invention;
Figures 3 to 5 show a rotation mechanism for use in the first or second embodiments of patient table; and
Figure 6 shows a suitable form of the rotateable elements.

### DETAILED DESCRIPTION OF THE EMBODIMENTS

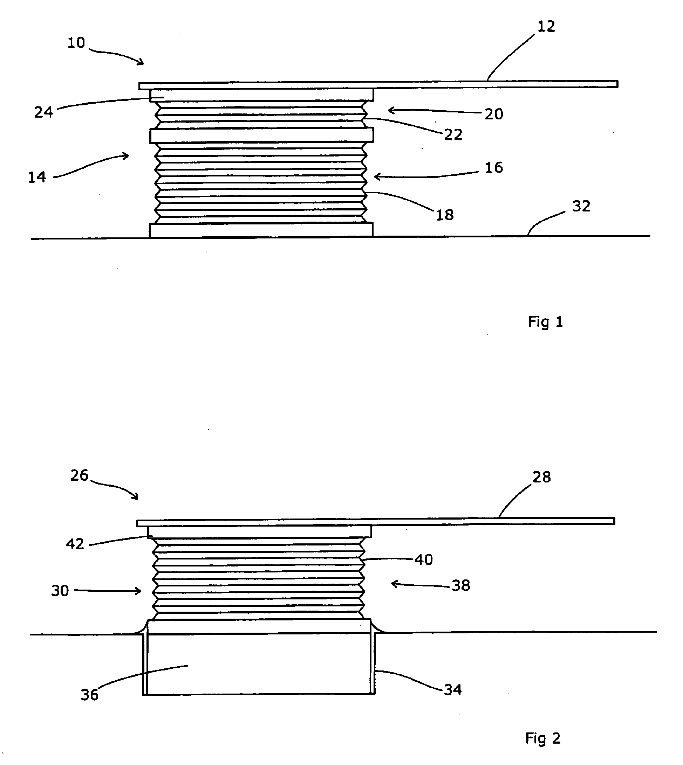

Figure 1 shows a patient support 10 in which a patient table 12 is provided for the patient to recline on. Beneath one end of the table 12 there is an adjustment mechanism 14 which, in this example, is provided in two sections. A lower section 16 includes a lifting mechanism which may be (for example) as disclosed in our earlier publication WO97/42876. To ensure the safety of the patient and the users, the mechanism is concealed within a series of bellows 18.

An upper section 20 includes a three-axis tilting and rotation mechanism as will be described herein, and is likewise concealed within a bellows 22 for safety reasons. The patient table 12 is then mounted to a translatable base 24 which includes lateral and longitudinal adjustment for the patient table 12 via suitable rack and pinion mechanisms. In this way, all six necessary degrees of freedom are permitted.

Figure 2 shows an alternative example of a patient support 26, in which a patient table 28 is again supported on an adjustment mechanism 30. In this case, however, instead of being placed directly onto the floor 32, the mechanism is seated in a recess 34 in the floor. The three-axis tilting mechanism 36 is concealed within the recess 34, and supports the lifting mechanism 38, again concealed via a bellows 40. Once again, the longitudinal and lateral adjustment mechanism 42 is sandwiched between the patient table 28 and the lifting mechanism 38. Arranging the mechanisms in this way, partly within the recess 34, allows the additional height of the three-axis tilting mechanism to be accommodated without affecting the overall height of the patient table.

Naturally, various features of these examples could be combined. For example, the order of various mechanisms as shown on Figure 1 could be combined with a recess 34 on Figure 2. Doing so may have some advantages, in that placing the tilting mechanism at the base will mean that there is some interdependence between the various adjustments; tilting the patient table 28 to one side by using the tilting mechanism at the base of the apparatus will mean that the patient table 28 will also move laterally in that direction.

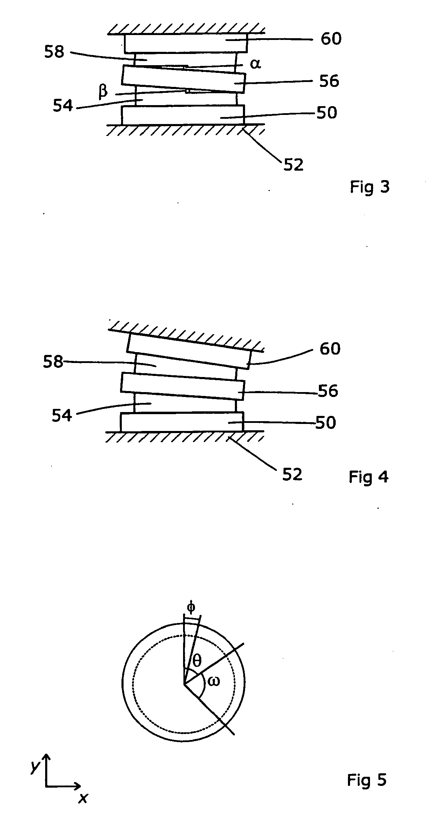

Figures 3, 4 and 5 show the 3-axis rotation mechanism. Figure 3 shows the mechanism at its "zero displacement" state, i.e. providing a surface that is flat and parallel to the ground. Figure 4 shows the apparatus at its maximum angular displacement in a chosen direction, and Figure 5 shows the mechanism from above.

Referring to Figure 3, the mechanism consists essentially of three rotateable elements with wedges between the first and second and the second and third in order to put the axis of the rotateable elements out of alignment. Thus, a first rotateable element 50 is supported directly on its base 52. The rotateable element 50 is such that it is able to rotate its upper face by a selected angle or a selected angular rate relative to its lower face. A suitable device for this is a torque motor (otherwise known as a rotary synchronous motor or a direct drive motor) as will be described later. Mounted to the top of the first rotateable element 50 is a wedge 54, which is generally cylindrical or disc-shaped, but has an upper face which is non-parallel to its lower face, deviating by an angle β. On the top of the first wedge 54, there is a second rotateable element 56 which may in form be identical to the first rotateable element 50. This then carries a second wedge 58 which, like the first wedge 54, has non-parallel upper and lower faces, deviating by an angle α. On the top of second wedge 58, there is a third rotateable element 60, which (again) maybe of the same form as the first and second rotateable elements 50, 56, and on top of the first rotateable element 60 there is a patient table or other item which needs to be adjusted.

As shown in Figure 3, the rotateable elements 50, 56 and 60 have been positioned so that the wedges 54, 58 are aligned oppositely. As the two wedges 54 and 58 are precisely oppositely arranged, in other words so that the tilt which they impose is directed in opposing directions, the stack as a whole remains upright with the lower and upper faces of the stack of rotateable elements 50, 56 and 60 being substantially parallel as both wedges are identical, ie α=β.

Figure 4 shows the result when at least the second rotateable element 56 is rotated by 180°, thereby placing the first and second wedges 54, 58 in the same orientation. A maximum tilt of a total angle of α+β is then imposed between the upper and lower faces of the complete stack.

A lesser tilt can be imposed by rotating the second rotateable element by less than 180°. If the second rotateable element 56 is rotated by such an amount, then the two wedges 54, 58 are placed at an intermediate position between the fully cancelled-out position of Figure 3 and the fully effective position of Figure 4. This means that the total angle of lean imposed by the stack is somewhere between the minimum shown in Figure 3 and the maximum shown in Figure 4. In this way, the amount as well as the direction of lean imposed by the mechanism can be controlled.

It can be seen the rotation of the first rotateable element 50 can then be used to determine the direction in which that angle of lean (α+β) is pointing. Finally, the third rotateable element 60 can be invoked to prevent any overall rotation around a vertical axis (in Figures 3-4) being imposed by the stack, or to choose the angle of such a rotation.

Thus, to summarise, the second rotateable element 56 creates an angle between the two wedges which sets the amount by which the top and bottom faces will be angled. The first rotateable element 50 (or the third rotateable element 60) determines the direction in which that angle will be imposed, and the third rotateable element 60 (or the first rotateable element 50) compensates for the vertical rotations imposed by the other two rotateable elements and/or imposes any required rotation without a vertical axis. In this way, the three rotateable elements allow rotation of the top surface of the device about any of the three axes of rotation.

Figure 5 shows the stack from above. For the three rotateable elements 50, 56 and 60 in the example shown in Figure 5, the angle θ is a rotation of the first rotateable element 50 and decides the direction in which the lean is to be imposed, this being the resultant of the rotations desired around the x and y axes shown in Figure 5. The angle ω is the angle of rotation of the second rotateable element 56, and is determined by the magnitude of that resultant and the angles α and β. The angle ϕ is the total desired rotation around the axis z perpendicular to the page in Figure 5, and the third rotateable element 60 is therefore rotated by an angle equal to (ϕ - θ - ω).

If the rotations are imposed one by one then there will be temporary transients in the position of a device such as a patient table 12 mounted on top of the stack. Thus, it will be better for the rotateable elements to be rotated simultaneously over a given time, so that their rate of rotation is set at the total rotation to be achieved divided by the given time. In this way, the stack will move smoothly from its current state to the desired state without any wild and undesirable swinging actions. A suitable control unit for the rotateable elements 50, 56, 60 can be provided to achieve this.

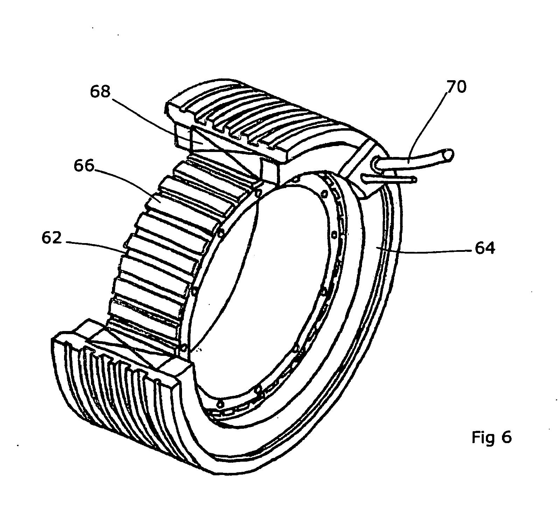

Figure 6 shows a suitable form of torque motor to provide the rotateable elements 50, 56, 60. This consists of a pair of concentric rings comprising an inner ring 62 and an outer ring 64. On the outer circumferential surface of the inner ring 60 there are a series of permanent magnets 66, and on the inner circumferential surface of the outer ring 64 there is a coil unit 68. An incoming cable 70 provides power to the coil unit 68 and allows it to be energised so that the magnetic interaction between this and the permanent magnets 66 then causes a relative rotation to be imposed between the inner and outer rings 62, 64. Assuming then that the outer ring 64 is fixed to a substrate, the inner ring 62 will then be caused to rotate. In this way, a compact disc-shaped rotateable unit is obtained.

It will of course be understood that many variations may be made to the above-described embodiment without departing from the scope of the present invention.

## Claims

1. A patient support, comprising a table supported via a three-dimensional rotation mechanism, the three-dimensional rotation mechanism comprising three rotateable elements fixed together in series, the first and second being fixed together with their rotational axes non-parallel, and the second and third being fixed together with their rotational axes non-parallel, the respective rotateable elements being connected via wedge elements having non-parallel faces.

2. A patient support according to claim 1 in which the rotateable elements are generally disc-shaped.

3. A patient support according to any one of the preceding claims in which rotateable elements are torque motors.

4. A patient support according to any one of the preceding claims in which the angle between the rotational axes of the first and second rotateable elements is substantially equal to the angle between the rotational axes of the second and third rotateable elements.

5. A patient support according to any one of the preceding claims, further comprising a control apparatus for controlling the first, second and third rotateable elements to each rotate by a specific angle of rotation.

6. A patient support according to claim 5 in which the control apparatus is adapted to rotate one of the first, second and third rotateable elements by an angle so as to substantially cancel the accumulated rotation of the remaining two of the first, second and third rotateable elements about the rotational axis of the first rotatable element.

7. A patient support according to claim 5 or claim 6 in which the control apparatus is adapted to rotate one of the first, second and third rotateable elements at a rate equal to the total rate of angular rotation of the remaining two of the first, second and third rotateable elements about the rotational axis of the first rotatable element but in an opposite direction.

8. A patient support according to claim 5 in which the control means is adapted to rotate two of the three rotateable elements by an angle or at a rate that is substantially equal.

9. A patient support according to claim 6 or 7 in which the control means is adapted to rotate the remaining two rotateable elements by an angle or at a rate that is substantially equal.

## Patentansprüche

1. Patiententräger mit einem Tisch, der durch einen dreidimensionalen Drehmechanismus getragen wird, wobei der dreidimensionale Drehmechanismus drei in Reihe miteinander verbundene drehbare Elemente aufweist, wobei das erste und das zweite mit ihren Drehachsen in nicht-paralleler Ausrichtung verbunden und das zweite und das dritte mit ihren Drehachsen in nicht-paralleler Ausrichtung miteinander verbunden sind, wobei die jeweiligen drehbaren Elemente über Keilelemente mit nicht-parallelen Oberflächen verbunden sind.

2. Patiententräger nach Anspruch 1, bei dem die drehbaren Elemente im Allgemeinen scheibenförmig sind.

3. Patiententräger nach einem der vorhergehenden Ansprüche, bei dem die drehbaren Elemente Torquemotoren sind.

4. Patiententräger nach einem der vorhergehenden Ansprüche, bei dem der Winkel zwischen den Drehachsen der ersten und zweiten drehbaren Elemente im Wesentlichen gleich dem Winkel zwischen den Drehachsen der zweiten und dritten drehbaren Elemente ist.

5. Patiententräger nach einem der vorhergehenden Ansprüche, der weiter eine Steuereinrichtung zum Steuern der ersten, zweiten und dritten drehbaren Elemente aufweist, um diese jeweils um einen spezifischen Drehwinkel zu drehen.

6. Patiententräger nach Anspruch 5, bei dem die Steuereinrichtung dazu eingerichtet ist, um eines der ersten, zweiten und dritten drehbaren Elemente um einen Winkel zu drehen, um so im Wesentlichen die kumulierte Drehung der übrigen zwei der ersten, zweiten und dritten drehbaren Elemente um die Drehachse des ersten drehbaren Elements aufzuheben.

7. Patiententräger nach Anspruch 5 oder Anspruch 6, bei dem die Steuereinrichtung dazu eingerichtet ist, eines der ersten, zweiten und dritten drehbaren Elemente mit einer Geschwindigkeit zu drehen, die gleich der Gesamtwinkelgeschwindigkeit der übrigen zwei der ersten, zweiten und dritten drehbaren Elemente um die Drehachse des ersten drehbaren Elementes, aber entgegengesetzt gerichtet ist.

8. Patiententräger nach Anspruch 5, bei dem die Steuereinrichtung dazu eingerichtet ist, zwei der drei drehbaren Elemente um einen Winkel oder mit einer Geschwindigkeit zu drehen, der oder die im Wesentlichen gleich ist.

9. Patiententräger nach Anspruch 6 oder 7, bei dem die Steuereinrichtung dazu eingerichtet ist, die übrigen zwei drehbaren Drehelemente um einen Winkel oder mit einer Geschwindigkeit zu drehen, der oder die im Wesentlichen gleich ist.

## Revendications

1. Support de patient comprenant une table supportée par un mécanisme de rotation en trois dimensions, le mécanisme de rotation en trois dimensions comprenant trois éléments rotatifs fixés ensemble en série, les premier et deuxième étant fixés ensemble avec leurs axes de rotation non parallèles, et les deuxième et troisième étant fixés ensemble avec leurs axes de rotation non parallèles, les éléments de rotation respectifs étant reliés par l'intermédiaire d'éléments en coin ayant des faces non parallèles.

2. Support de patient selon la revendication 1, dans lequel les éléments rotatifs ont une forme générale de disque.

3. Support de patient selon l'une quelconque des revendications précédentes, dans lequel les éléments rotatifs sont des moteurs couple.

4. Support de patient selon l'une quelconque des revendications précédentes, dans lequel l'angle entre les axes de rotation des premier et deuxième éléments rotatifs est substantiellement égal à l'angle entre les axes de rotation des deuxième et troisième éléments rotatifs.

5. Support de patient selon l'une quelconque des revendications précédentes, comprenant en outre un dispositif de commande pour commander les premier, deuxième et troisième éléments rotatifs afin de les faire tourner chacun selon un angle de rotation spécifique.

6. Support de patient selon la revendication 5, dans lequel le dispositif de commande est adapté pour faire tourner l'un des premier, deuxième et troisième éléments rotatifs selon un angle afin d'annuler substantiellement la rotation accumulée des deux éléments restants parmi les premier, deuxième et troisième éléments rotatifs autour de l'axe de rotation du premier élément rotatif.

7. Support de patient selon la revendication 5 ou 6, dans lequel le dispositif de commande est adapté pour faire tourner l'un des premier, deuxième et troisième éléments rotatifs à une vitesse égale à la vitesse totale de rotation angulaire des deux éléments restants parmi les premier, deuxième et troisième éléments rotatifs autour de l'axe de rotation du premier élément rotatif mais dans une direction opposée.

8. Support de patient selon la revendication 5, dans lequel le moyen de commande est adapté pour faire tourner deux des trois éléments rotatifs selon un angle ou à une vitesse qui est substantiellement égale.

9. Support de patient selon la revendication 6 ou 7, dans lequel le moyen de commande est adapté pour faire tourner les deux éléments rotatifs restants selon un angle ou à une vitesse qui est substantiellement égale.
